# EUROPEAN PATENT SPECIFICATION

(11) **EP 3 020 424 B1**
(45) Date of publication and mention of the grant of the patent: **14.11.2018**
(21) Application number: 14822293.8
(22) Date of filing: 03.07.2014
(51) Int. Cl.: A61L 27/44, A61L 27/46, A61L 27/58

(54) **BONE DEFECT FILLING MATERIAL, AND PRODUCTION METHOD THEREFOR**
KNOCHENDEFEKTFÜLLMATERIAL UND HERSTELLUNGSVERFAHREN DAFÜR
MATÉRIAU DE REMPLISSAGE D'UNE PORTION D'OS DÉFECTUEUSE, ET PROCÉDÉ DE PRODUCTION DUDIT MATÉRIAU

(30) Priority: 09.07.2013 US 201361844305 P; 29.01.2014 JP 2014014146
(43) Date of publication of application: 18.05.2016
(60) Divisional application: 17191860.0
(73) Proprietor: National University Corporation Nagoya Institute Of Technology, Nagoya-shi, Aichi 466-0061 (JP); Orthorebirth Co.Ltd., Kanagawa 224-0033 (JP)
(72) Inventor: KASUGA, Toshihiro, Nagoya-shi Aichi 466-0061 (JP); MAKITA, Masashi, Yokohama-shi Kanagawa 224-0033 (JP)
(74) Representative: Provvisionato, Paolo
(86) International application number: PCT/JP2014/067742
(87) International publication number: WO 2015/005205

(56) References cited:
- WO-A2-2012/154842
- JP-A- 2010 522 620
- JP-B2- 5 179 124
- US-A1- 2010 119 564
- US-A1- 2012 315 319
- MCCULLEN S D ET AL: "Electrospun composite poly(L-lactic acid)/tricalcium phosphate scaffolds induce proliferation and osteogenic differentiation of human adipose-derived stem cells.", BIOMEDICAL MATERIALS, INSTITUTE OF PHYSICS PUBLISHING, BRISTOL, GB, vol. 4, no. 3, 2009, page 35002, XP020162991, ISSN: 1748-605X
- TAKASHI WAKITA ET AL: "Effect of preparation route on the degradation behavior and ion releasability of siloxane-poly(lactic acid)-vaterite hybrid nonwoven fabrics for guided bone regeneration", DENTAL MATERIALS JOURNAL, vol. 30, no. 2, 1 January 2011 (2011-01-01), pages 232-238, XP055258993, JP ISSN: 0287-4547, DOI: 10.4012/dmj.2010-141
- S D MCCULLEN ET AL.: 'Electrospun composite poly (L-lactic acid)/tricalcium phosphate scaffolds induce proliferation and osteogenic differentiation of human adipose-derived stem cells' BIOMEDICAL MATERIALS vol. 4, no. 3, 2009, pages 035002/1 - 035002/9, XP020162991

## Description

### Technical Field

The present invention relates to a method of producing a material for filling a bone defect which is formed of biodegradable fibers in a cotton-like structure.

### Background Art

Recently, materials for filling a bone defect of a type which can rebuild the bone of a defect portion by utilizing a self-regenerating ability of bone has been developed. The bone filling material of this type for filling a bone defect promotes osteogenesis by osteocyte by supplying bone formation factor by implanting porous fibrous material containing ceramic which works as a bone formation factor.

The above-mentioned type of the material for filling a bone defect is produced by producing fibers by electrospinning or other method from a spinning solution which is produced by mixing a solution of a biodegradable polymer, such as poly L lactic acid (PLLA) or polylactic acid-polyglycolic acid copolymer (PLGA). After the material i s implanted in a body, the matrix polymer of the biodegradable fiber works as a scaffold to maintain the three dimensional skeleton of the material in a defect portion. And, as the polymer is gradually absorbed and decomposed by contacting with biological fluids, bone forming factors, such as calcium phosphate, are exposed or released, and perform the biological activities of bone formation. Then, after the bone formation has been completed, the biodegradable polymer disappears by being decomposed and absorbed completely in the living body.

As a ceramic to be used as bone forming factors, bioabsorbable calcium phosphate, such as β-tricalcium phosphate (β-TCP), is used as a material having both biocompatibility and osteoconductivity. The mechanism of the biological activities of bioabsorbable calcium phosphate is not necessarily clear. However, it is thought that in a bone defect portion, bone forming cells attach well to the surface of calcium phosphate and proliferates and differentiates thereon, thereby becoming a scaffold (scaffold or substrate) for bone formation. It is known that calcium carbonate also shows the a function of attaching bone cell and proliferation.

While it is known that a bone is formed by a remodeling that is caused by coupling of osteoclasts and osteoblasts, it is experimentally confirmed and reported that if a small amount of silicon is supplied together with calcium during the above process, proliferation of osteoblasts is stimulated, and proliferation and differentiation are promoted. Based on above knowledge and understanding, a material for filling a bone defect in which a biodegradable polymer containing silicon-releasing vaterite phase calcium carbonate (SiV) particles has been proposed as a new type material for filling a bone defect (Patent literature 1). After the material is filled in a bone defect and is in contact with body fluids, a small amount of silicon is released gradually and stimulates osteoblasts as the calcium carbonate is being dissolved, thereby promoting proliferation and differentiation. Further, calcium ions released by decomposition of calcium carbonate are supplied to the vicinity of cells, whereby the activity of the cells is activated and high bioactivity is realized.

### Prior art references

### Patent Literature

Patent literature 1: Japanese Patent No. 5179124 (US2010119564)

### Non Patent Literature

Non patent literature 1: Walsh et al. β-TCP bone graft substitutes in a bilateral rabbit tibial defect model. Biomaterials 29 (2008) 266-271)
Non Patent Literature 2: Obata et al. Electrospun microfiber meshes of silicon-doped vaterite / poly(lactic acid) hybrid for guided bone regeneration. Acta Biometatialla 6 (2010) 1248-1257.
Non patent Literature 3: Fujiwara et al. Guided bone regeneration membrane made of polycaprolactone / calcium carbonate composite nano-fibers. Biomaterials 26 (2005) 4139-4147).
Non patent literature 4: Hench LL. Polak JM: Third-generation biomedical materials. Science 2002, 295: 1014 -1017) US2012315319 discloses bone regeneration material with electrospun fibers prepared from a composite of poly(lactic acid) and silicon-releasing calcium carbonate.

### Summary of Invention

### Problem to be solved by the invention

Rebuilding a lost bone by utilizing the self-regenerating ability of the bone is an excellent method by which permanent bone repair can be achieved. However, the self-regeneration of a bone needs a long period of time of at least three to six months after a material has been implanted. Therefore, the material for filling a bone defect used for such a method needs to initiate a bone regenerating activity as soon as possible after it was implanted, and also continue the activity of promoting bone formation by remaining in the defect portion until sufficient bone formation is achieved. However, until now, there has not been obtained any material for filling a bone defect that satisfies these contradicting requirements.

### Means to solve the problem

The scope of the invention is defined by the claims.

Any references in the description to methods of treatment refer to the compositions for use in a method for treatment of the human (or animal) body by therapy. The material for filing a bone defect is a material for filling a bone defect that includes biodegradable fibers produced by electrospinning in a cotton-like structure, and the biodegradable fibers contain calcium phosphate particles in an amount of 40% to 60% by weight, preferably 40% by weight, calcium carbonate particles in an amount of 10% by weight or more, preferably 30% by weight, and preferably a poly-L-lactic acid polymer in an amount of 30% by weight or more, preferably 30% by weight or all the remainder. Further, amount of an amorphous phase of the poly-L-lactic acid polymer is 75% to 98%, preferably 85% to 95%, more preferably 88% to 92%.

Because Polymer content of the biodegradable fibers used for the material for filling a bone defect is limited as small as possible as far as fibers can be spun by electrospinning, exposure of calcium phosphate particles and calcium carbonate particles on the surface of a fiber is large, and the area which directly contacts with body fluids is large. As a result, high biological activity is achieved from the particles of calcium phosphate and the calcium carbonate.

The calcium carbonate contained in the material for filling a bone defect is preferably a silicon-releasing calcium carbonate of a vaterite phase. Because such silicon-releasing calcium carbonate has a fast dissolution rate, calcium ions are released early after being implanted and create a calcium rich environment. On the other hand, silicon species doped in the calcium carbonate are released gradually and stimulate proliferation of osteoblasts and promotes bone formation.

The material for filling a bone defect induces generation of bone-like apatite on a surface of a fiber by releasing a rich amount of calcium ions from the calcium carbonate. Polylactic acid which is a matrix polymer of the fiber has many carboxyl groups, and the polylactic acid is hydrolyzed by contacting with biological fluids, thereby forming a carboxyl group which induces nucleation of bone-like apatite

As the calcium carbonate of the material for filling a bone defect calcium carbonate of vaterite phase is preferably used. Generally, based on the difference of crystal structure, calcium carbonate is classified into three types: a calcite phase, an aragonite phase, and a vaterite phase. Calcium carbonate of a vaterite phase has the highest solubility in the biological fluid of a human body. Therefore, PLA containing vaterite phase calcium carbonate has a high bone-like apatite forming ability.

Bioabsorbable calcium phosphate used for the material for filling a bone defect is bioabsorbed slowly over time after being implanted in a defect and bone replaced. Because the material for filling a bone defect contains 40% or more of bioabsorbable calcium phosphate, bone formation by absorption and replacement is performed effectively.

Biodegradable polymer used for the material for filling a bone defect remains in a defect portion while maintaining a skeleton structure until calcium phosphate is absorbed and bone replaced, and works as a scaffold where bone cells perform their activity during formation of the bone. Because PLLA is not easily hydrolyzed, the concern that PLLA will disappear immediately after implantation by being decomposed and absorbed upon contacting with body fluid is small.

Outer diameter of the biodegradable fibers of the material for filling a bone defect is preferably from 10 to 50 µm, more preferably from 30 to 50 µm.

A method of producing a material for filling a bone defect includes the steps of: providing a mixture of calcium phosphate particles and SiV particles in a melted polymer solution in a kneader such that weight ratio of the three components are 40% to 60% by weight of calcium phosphate, 10% by weight or more of silicon-releasing calcium carbonate, and remainder is 30% by weight or more of poly L lactic acid; kneading the components in that state; cooling and solidifying the kneaded mixture to produce a composite body in which the molecular weight of the polymer is 200,000 to 250,000 and the amount of amorphous phase of the polymer is 75% or more, preferably 85% or more; producing a spinning solution by dissolving the composite by using a solvent; producing biodegradable fibers by spinning the spinning solution by using an electrospinning method; and producing the material for filling a bone defect i n a cotton-like structure by receiving the biodegradable fibers in a collector filled with ethanol and accumulating the biodegradable fibers thereon.

The method of producing the material for filling a bone defect includes the steps of kneading a solution containing silicon-releasing calcium carbonate particles, calcium phosphate particles, and melted poly lactic acid in a predetermined amounts respectively for a predetermined time at a predetermined temperature in a kneader by using the kneader; and during this process amino group portion of siloxane contained in the silicon-releasing calcium carbonate particles and a carboxy group at an end of the poly(lactic acid) structure is bonded (amino bonding). By using this process, the orderly structure of polylactic acid contained in the spinning solution is disturbed and the ratio of an amorphous phase of the polylactic acid becomes high, and solubility increases. As a result, the material for filling a bone defect produced by electrospinning method by using the spinning solution thus produced has a higher absorptivity in a living body. The amount of amorphous phase in the poly L lactic acid of the material for filling bone defect of the present invention is preferably from 75% to 98%, more preferably from 85% to 95%, further more preferably from 88% to 98%.

In the material for filling a bone defect approximately spherical TCP particles ( average particle diameter is from 3 to 4 µm) and approximately spherical SiV particles ( average particle diameter is about 1 µm) are dispersed almost homogeneously in a matrix polymer i n the composite fiber having a diameter of about 10 to 50 µm produced by electrospinning. Preferably, both the TCP particles and the SiV particles are dispersed almost homogeneously in the matrix polymer without being unevenly distributed in a specific portion. As a result, minute TCP particles and SiV particles are homogeneously dispersed widely near the surface of a fiber and over the vicinity of the center of the fiber. Because of that, after the material has been filled in a bone defect, as the biological absorption of the polymer proceeds, bone resorption of the TCP particles and silicon releasing from the SiV occurs uniformly in the bone defect portion for a comparatively long period of time.

### Brief Description of Drawings

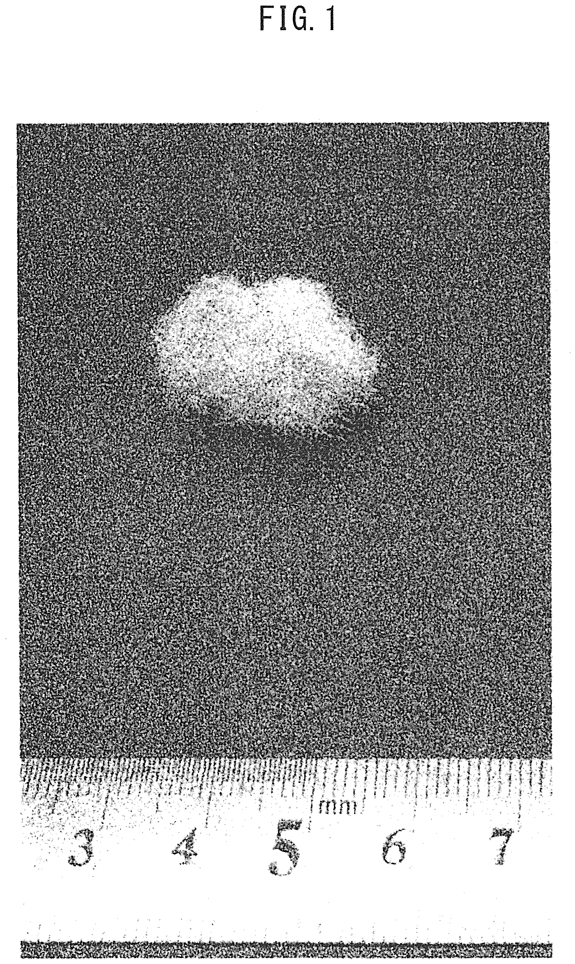

[Fig. 1] Fig. 1 shows a general-view photograph of the material for filling a bone defect in an embodiment.
[Fig. 2] Fig. 2 is a SEM photograph showing a surface of a fiber of a material for filling a bone defect in an embodiment.
[Fig. 3] Fig. 3 is a SEM photograph showing a cross section of the fiber of a material for filling a bone defect in an embodiment.
[Fig. 4] Fig. 4 is a SEM photograph showing a state of the fibers entangled each other forming a cotton-like structure of a material for filling a bone defect in an embodiment.

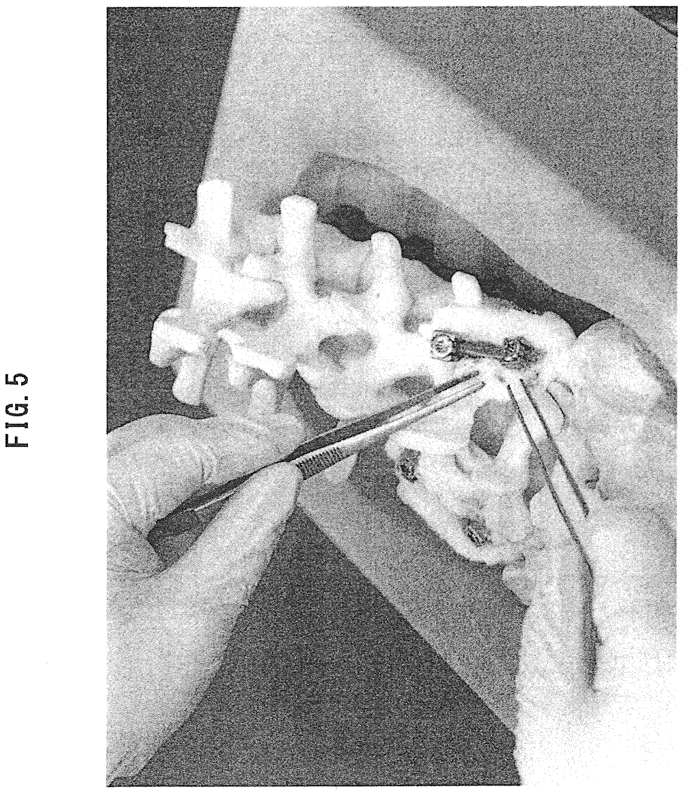

[Fig. 5] Fig. 5 shows a method of using the material for filling a bone defect in a cotton-like structure in an embodiment in which the material is implanted in a vicinity of an implant device for fixing a spine of a human body.
[Fig. 6] Fig. 6 shows a method of using the a material for filling a bone defect in which autologous is wrapped by the material in cotton-like structure in an embodiment.

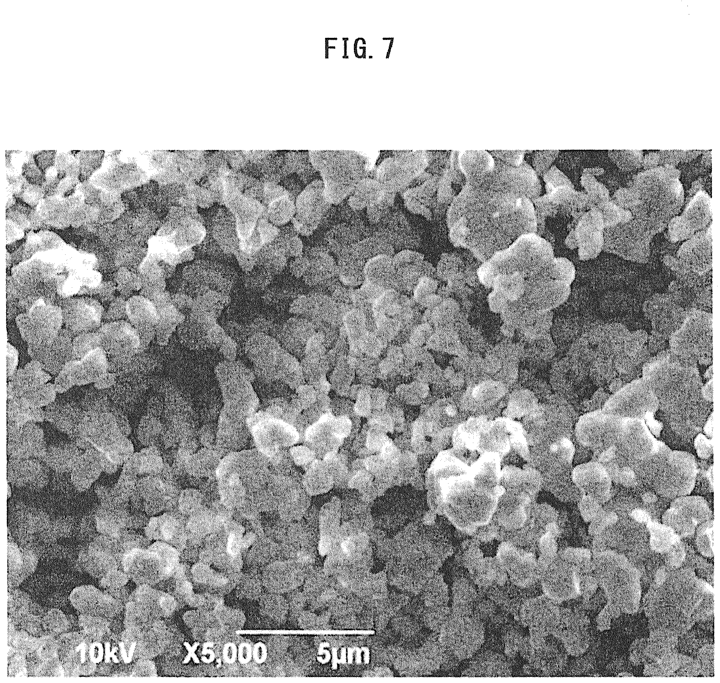

[Fig. 7] Fig. 7 is a SEM photograph of β-TCP particles used for the material for filling a bone defect in an embodiment of the present invention.
[Fig. 8] Fig. 8 is a SEM photograph of silicon-releasing calcium carbonate (SiV) used for the material for filling a bone defect in an embodiment of the present invention.
[Fig. 9] Fig. 9 is an imaginary structure of the silicon-releasing calcium carbonate used for the material for filling a bone defect in an embodiment of the present invention.
[Fig. 10] Fig. 10 is a graph showing releasing characteristic of Si and Ca when silicon-releasing calcium carbonate is immersed in a tris buffer solution.

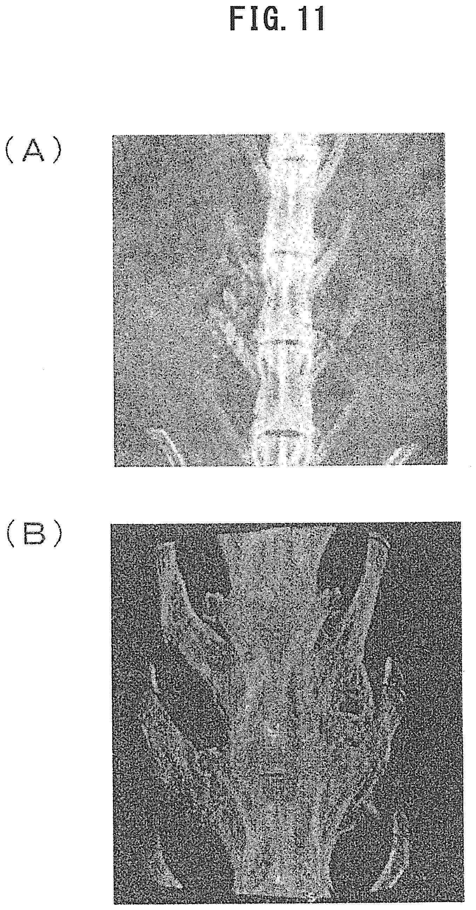

[Fig. 11] Fig. 11 (A) is an X-ray image showing a state immediately after the material for filling a bone defect of the cotton-like structure of an embodiment is implanted in a spine of a rabbit. The right-hand side of the spine shows a state where the cotton was implanted alone, and the left-hand side of the spine shows a state where the cotton was implanted mixed together with an autologous bone. Fig. 11 (B) is a CT image after twelve weeks passed from the state shown in Fig. 11 (A). The left-hand side of the spine shows a state where the cotton was implanted alone, and the right-hand side of the spine shows a state where the cotton was mixed together with an autologous bone.
[Fig. 12] Figs. 12(A) and 12(B) are dye slice images showing a state after twelve weeks after a material for filling a bone defect in an embodiment has been implanted in a femur of a rabbit together with bone aspirate (Bone Marrow Aspirate).
[Fig. 13] Figs. 13(A), 13(B), and 13(C) are dye slice images showing a state of after twelve weeks after a material for filling a bone defect in an embodiment has been implanted into a spine of a rabbit together with bone aspirate (Bone Marrow Aspirate).
[Fig. 14] Figs. 14(1) - (5) are photographs showing a change of an appearance due to the elapse of 1 - 14 days after samples [1] to [5] have been immersed in hydroxide solutions respectively.
[Fig. 15] Fig. 15 is a graph showing a change of a molecular weight of PLLA due to elapse of 1 - 14 days after samples [1] to [4] have been immersed in sodium hydroxide solutions. Depending on polylactic acid content and SiV content, difference of molecular weight before the immersion was observed. Result was that molecular weight largely decreased immediately after immersing the samples, and thereafter, the molecular weight decreased gradually.
[Fig. 16] Fig. 16 is a graph showing a change of a dry weight of the cotton like material due to the elapse of 1 - 14 days after samples [1] to [5] have been immersed in sodium hydroxide solutions. In the samples after the immersion, as a trend, the lower the molecular weight of PLLA was, the larger the decrease of the weight was.
[Fig. 17] Figs. 17(1) and (2) show the results of DSC measurement which measured the crystallinity of the samples [1] to [5].
[Fig. 18] Figs. 18(1) and (2) show the results of DSC measurement for another sample [2]' (70SiV-30PLLA), [3]' (30SiV-40TCP-30PLLA) and [4]' (10SiV-60TCP-30PLLA) which were produced by the same method as that of the samples of Fig. 17.
[Fig. 19] Fig. 19 shows a decrease of the molecular weight of PLLA in the case where a material for filling a bone defect in an embodiment has been subjected to sterilization treatment by being irradiated with 35 kGy of γ rays.

### Description of Embodiments

Hereinafter, embodiments of the present invention will be described in detail with reference to the drawings.

### <Biodegradable polymer>

As a biodegradable polymer of a material for filling a bone defect poly L lactic acid (hereafter referred to as poly L lactic acid or PLLA) may be preferably used. Although PLLA is bioabsorbable, PLLA is more difficult to be hydrolyzed as compared to PLGA. Therefore, the biodegradable fiber formed of PLLA as a matrix polymer does not decompose easily when it is contacted with body fluids at a defect portion, and the biodegradable fiber remains for a long period of time without disappearing so that the skeleton of the material can be maintained.

On the other hand, in order for the bone growing factors contained in the matrix polymer, such as calcium phosphate and calcium carbonate to perform biological activities, these fine particles need to contact with body fluids. If the matrix polymer does not dissolve in human body fluids easily, bone forming factors may be prevented by the matrix polymer from performing sufficient osteogenic effect.

Because PLGA is easily decomposed and absorbed upon contacting with fluids, PLGA less prevents the bone forming factors contained therein from directly contacting with the biological fluids. However, because the decomposition/absorption rate of PLGA is fast, the skeleton of the material cannot be maintained for a long period of time to make a scaffold for bone formation. Because the rate of decomposition of PLLA when it contacted with biological fluids is considerably slow, PLLA remains in a body for a long period of time after being implanted in the body. Therefore, the problem that PLLA disappears before sufficient bone formation is completed is few. Conversely, because PLLA is not easily decomposed nor absorbed, there is a possibility that PLLA prevents bone forming factors contained therein from being exposed to the biological fluids or eluted outside. Further, even after the bone formation is completed, it is not desirable for the health of human body that PLLA remains in the body for a long period of time without disappearing.

If a melted PLA is mixed with silicon-releasing calcium carbonate (SiV) by kneading using the kneader, molecular weight of the PLA decreases. During that process of heat kneading, partial reaction occurs such that a bonding (an amide bond) takes place between an amino group portion of siloxane and a carboxy group at an end of a polylactic acid structure (Wakita et al, Dental Materials Journal 2011; 30(2): 232-238). Because of this, orderly structure of polylactic acid is disturbed and the ratio of amorphous phase of the polylactic acid becomes higher, which causes increase of solubility and fast absorption in a living body. However, in order to form a bone, it is desirable that a material itself is not absorbed and does not disappear for at least three to six months so that activity place for cells is secured. Because β-TCP does not have a silicic acid portion that is coupled to an amino group like that of SiV, heat kneading does not easily cause a change to PLA and, thus absorptivity of PLA is not likely to become high rapidly.

Inventors of the present invention found that, by mixing a substantial amount of calcium phosphate having no silicic acid portion coupled to amino group with a composite of SiV and PLLA, bio-absorptivity of the composite material becomes slower than that of the composite of SiV and PLLA. Therefore, it is possible to control the absorptivity of the composite material such that the composite material does not disappear before a bone is formed therein.

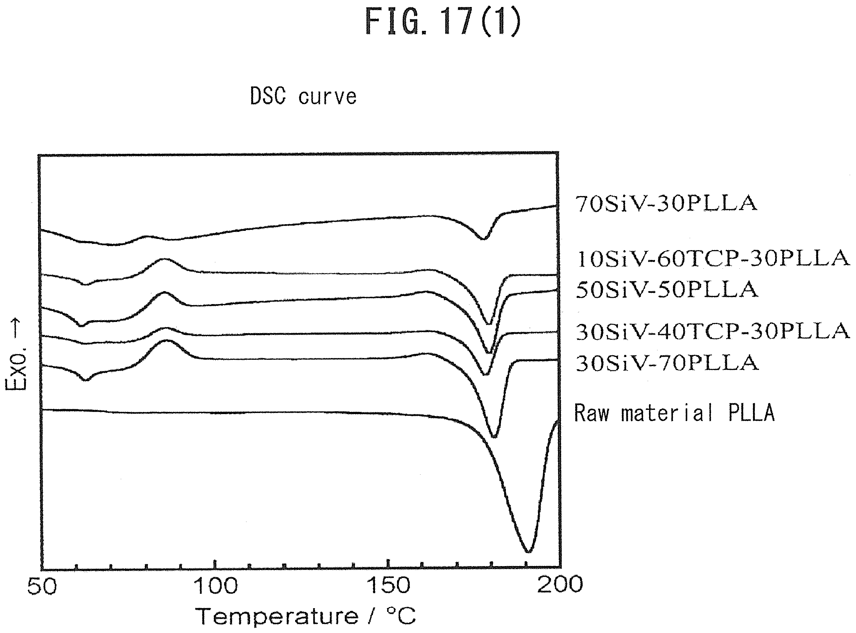

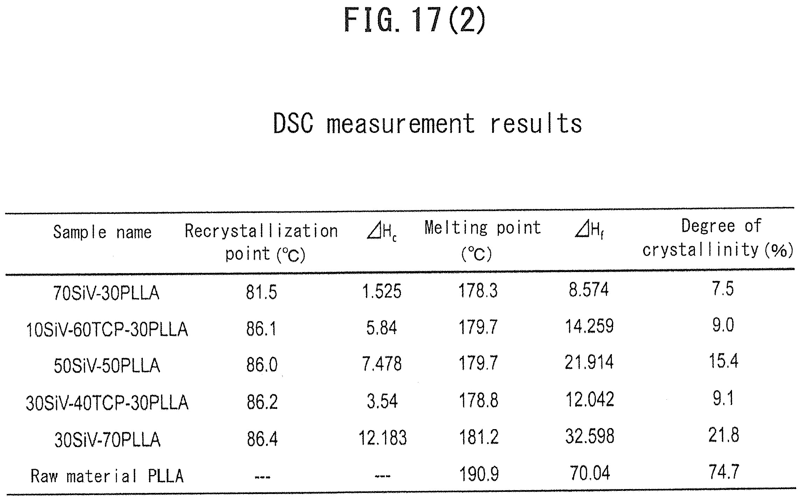

It is thought that other than a blending ratio of calcium carbonate and calcium phosphate at the time of kneading, ratio of amorphous phase of poly L lactic acid in the material for filling a bone defect is greatly influenced by an amount of poly L lactic acid contained in a fiber. In the embodiment shown in Fig. 17, in a sample [1] which contains 70% by weight of poly L lactic acid to 30% by weight of silicon-releasing calcium carbonate, the crystallinity of the poly L lactic acid is 21.8%. On the other hand, in a sample [1] which contains 30% by weight of poly L lactic acid to 70% by weight of silicon-releasing calcium carbonate, the crystallinity of the poly L lactic acid is greatly lowered to 7.5%.

### <Calcium phosphate>

Calcium phosphate used for the material for filling a bone defect may include bioabsorbable calcium phosphate, such as calcium hydrogenphosphate, octacalcium phosphate, tetracalcium phosphate, tricalcium phosphate, and carbonic acid containing apatite. β-tricalcium phosphate is especially suitable as a material to make a scaffold for proliferation and differentiation of cells of an osteoblast system. Its appearance is powder-like. The average diameter of particle constituting the powder is 3-4 µm. In consideration of the fact that the outer diameter of a fiber constituting a filling material of the present invention is 10 to 50 µm, a particle diameter of 6 µm or less is preferable. The average diameter of the silicon-releasing calcium carbonate particles is about 1 µm.

### <Silicon-releasing calcium carbonate (SiV)>

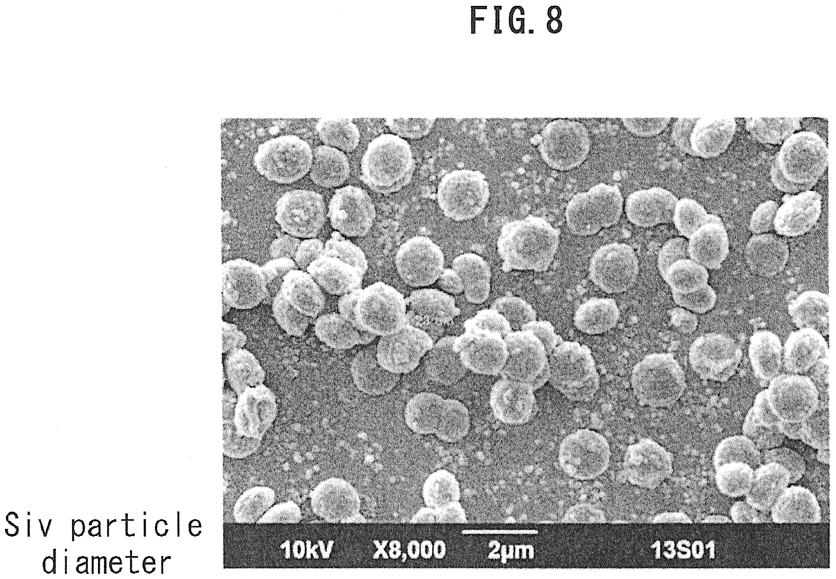

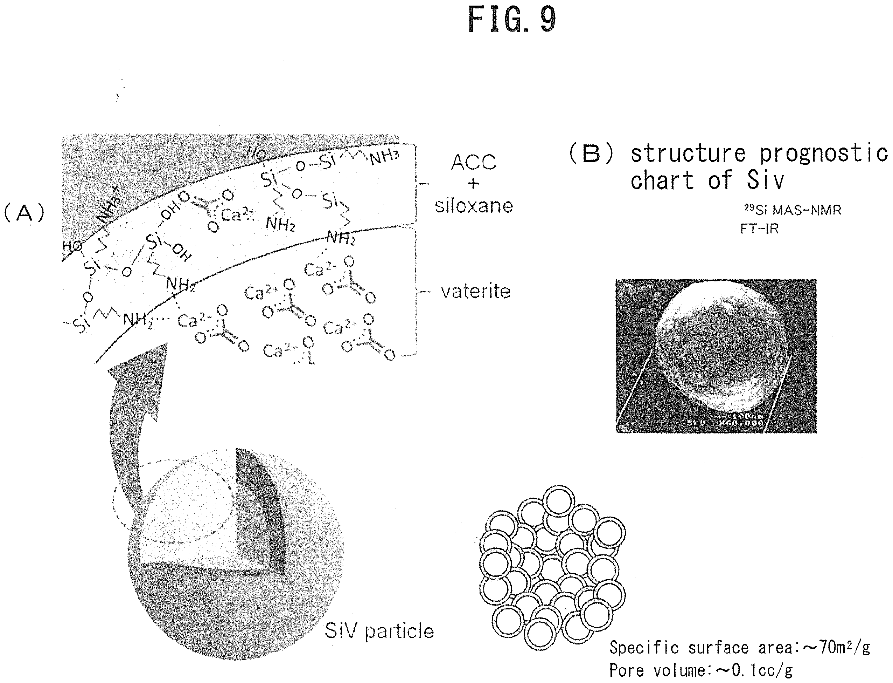

SiV used for the material for filling a bone defect is a composite of siloxane and calcium carbonate, its appearance is powder-like, and the diameter of particles constituting the powder is about 1 µm. Fig. 9 shows an imaginary structure of SiV. Fig. 8 is a photograph by a scanning electron microscope (SEM). Production method of SiV is disclosed in detail in Japanese Unexamined Patent Publication No. 2008-100878 (Silicon elution calcium carbonate and production method of it). Silicon content in SiV is 2% to 4% by weight, preferably 2% to 3% by weight. If silicon content exceeds 4% by weight, Siv does not become spherical but becomes an indeterminate form, which may cause uneven dispersion of the particles in PLLA, and thus undesirable.

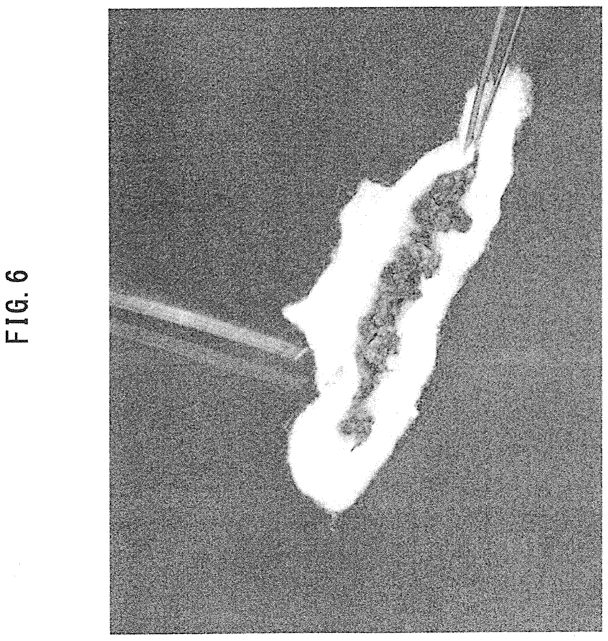

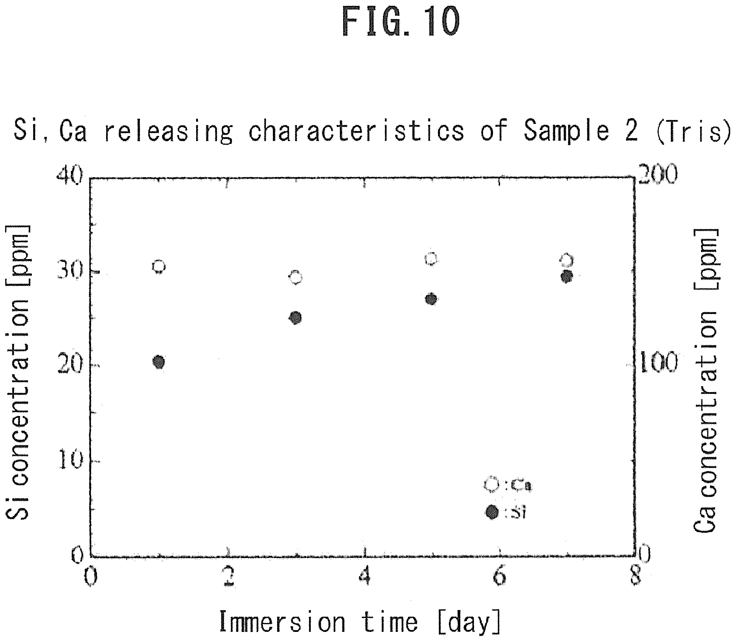

If SiV is implanted in a defect part and contacted with body fluids, vaterite phase calcium carbonate is hydrolyzed, and calcium ions are released in a short period of time. Silicon is gradually released. In Japanese Patent Application No. 2011-021790 filed before the present application, the inventors of the present invention disclosed releasing characteristics of calcium ions and silicon species in silicon-releasing calcium carbonate. PLLA in an amount of 42 g and 18 g of 2SiV (vaterite phase calcium carbonate which contains 2% by weight of Si) were heated at 200 °C for 45 minutes by using a heating kneader to obtain a composite containing 30% by weight of 2SiV. A spinning solution was prepared by mixing 9.3 g of CHCl₃ with 1 g of the composite. By using this spinning solution, a cotton-like material was produced by an electrospinning method. The obtained cotton-like material was immersed in a tris buffer solution and was made to stand still in an incubator held at 37 °C, and then, after having been immersed for a predetermined period, the solution was subjected to solid-liquid separation. Subsequently, the concentration of Si and Ca in the liquid was measured with an induction plasma-coupled spectrographic analysis (ICP). Fig. 10 is Fig. 6 of Japanese Patent Application No. 2011-021790 and shows the releasing characteristics when Si and Ca are immersed in a tris buffer solution. Fig. 10 shows a situation that, after having been immersed in the tris buffer solution, a large quantity of calcium is released within one day, and thereafter, a very small amount of silicon is gradually released with the lapse of time.

### <Production of a material for filling a bone defect>

Mixture of calcium phosphate particles and calcium carbonate particles is added to a biodegradable polymer melt that was produced by heating the polymer at a high temperature in a kneader, and then mixed and kneaded therein, and thereafter cooled under a room temperature to be solidified. Then, a composite body of silicon-releasing calcium carbonate, calcium phosphate, and biodegradable polymer is produced. The weight ratio of the three components is made such that the PLLA is 30% by weight or more, the calcium phosphate is 40% to 60% by weight, and the silicon-releasing calcium carbonate is 10% by weight or more. More preferably, the PLLA is 30% by weight, the calcium phosphate is 40% by weight, and the silicon-releasing calcium carbonate is 30% by weight.

Next, a spinning solution is produced by dissolving the composite by chloroform. The spinning solution is spun by using an electrospinning method under a certain method/condition to produce a cotton-like material formed of biodegradable fibers. A collector container is filled with an ethanol liquid so that the electrospun fibers are received by the liquid and the electrospun fibers are accumulated in the collector container. The ethanol liquid filled in the collector container removes the chloroform remaining on a surface of fibers. As a result, it becomes possible to prevent fibers deposited on the collector plate from adhering each other, thereby forming a cotton-like material which has soft light feeling with low bulk density.

In order to promote the bone formation, it is desirable that content of inorganic particle (SiV, β-TCP) contained in the composite is high, because biological activities is increased. However, if the inorganic particles are increased beyond a certain limit, it becomes difficult to knead the particles with polymer. In an experiment conducted by the inventors of the present invention, kneading could not be conducted with 80% by weight of the entire inorganic particles and 20% by weight of PLLA. In the material for filling a bone defect of the present invention, it is preferable that PLLA content is 30% by weight or more and 40% by weight or less, and the remainder is constituted by bone forming inorganic ceramic particles (SiV, calcium phosphate).

The spinning solution of the electrospinning of the present invention is produced through the following two steps. In the first step, a solution produced by mixing inorganic particles to polymer melted at high temperature is kneaded in a kneader at a certain temperature for a certain time, and cooled and solidified so as to produce a composite. In the next step, the produced composite is dissolved by chloroform to produce the spinning solution.

Because PLLA has a highly orderly molecular arrangement, it is difficult to hydrolyze even if it is contacted with a body fluid. In order to produce a spinning solution, a PLLA melt is kneaded by using a kneader. In the mixing process (kneading while applying heat), it partially reacts with SiV particles such that bonding (an amide bond) takes place between an amino group portion of siloxane contained in SiV and a carboxy group at an end of polylactic acid (Wakita et al, Dental Materials Journal 2011; 30(2): 232-238). Accordingly, the orderly arrangement the polylactic acid is disturbed. As a result, a ratio of an amorphous phase of the polylactic acid becomes high, solubility of the material increases. Contrary, if inorganic material added to PLLA does not make amide bonds with polylactic acid, a ratio of an amorphous phase in the PLLA is not increased. Therefore, solubility does not become high rapidly.

In the material for filling a bone defect, since kneading is performed with a blending ratio of 40% to 50% by weight of calcium phosphate, 10% by weight or more of silicon-releasing calcium carbonate, and the remainder of 30% by weight or more of PLLA, a ratio of amorphous in biodegradable fibers is controlled appropriately. As a result, the solubility of the PLLA matrix polymer to body fluids is controlled appropriately. In an embodiment shown in Fig. 17, the crystallinity of a sample [2] which was prepared by adding 30% by weight of PLLA to 70% by weight of SiV is 8% or less. In sample [3] (30SiV-40TCP-30PLLA) and sample [4] (10SiV-60TCP-30PLLA) which was prepared by reducing a certain amount of SiV and adding a certain amount of TCP and reducing corresponding amount of SiV, crystallinity of PLLA became as high as from 8% to 15%.

Outer diameter of the biodegradable fibers of the material for filling a bone defect produced by using electrospinning is preferably 10 to 50 µm, more preferably 30 to 50 um. In the spinning by electrospinning, outer diameter of a fiber generally tends to become several µm or less. As compared with it, the biodegradable fiber of the material for filling a bone defect is thick. By making the outer diameter of a fiber to be 10 µm or more, it becomes possible to create a space (gap) between the fibers which is necessary for cells to enter into the inside of the cotton-like porous body of the present invention. It is difficult to make the outer diameter of a fiber spun by using electrospinning to be 50 µm or more.

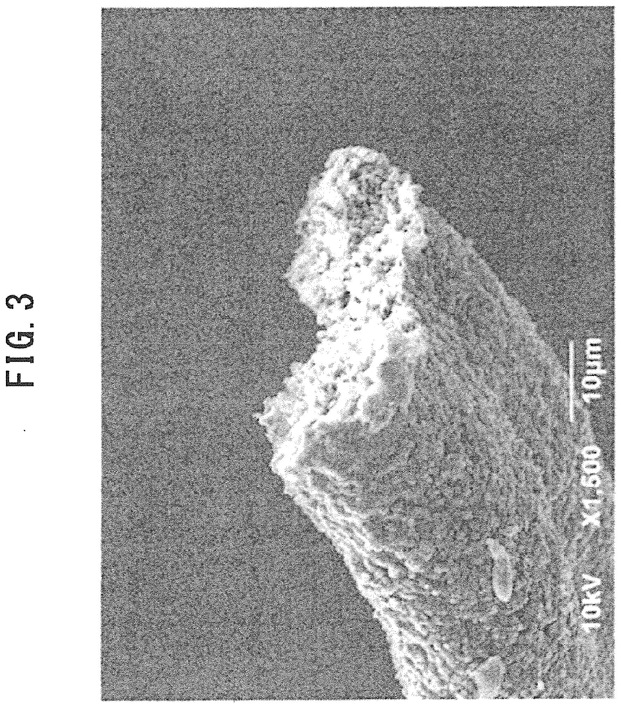

As shown in Fig. 3, innumerable ultrafine pores are formed on the surface of a fiber of the biodegradable fibers of the material for filling a bone defect. In the spinning by electrospinning, ultrafine pores are formed on a surface of a fiber during the process in which a spinning solution emitted in a form of fiber from a nozzle is evaporated. In the material for filling a bone defect, it is assumed that ultrafine pores formed on biodegradable fibers greatly increase the area of contact between contained ceramic particles (bone formation factors) and body fluid.

### <Sterilization treatment>

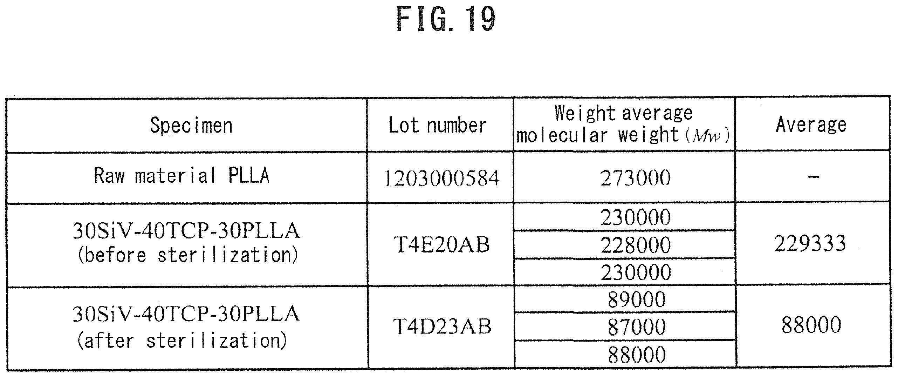

After the material for filling a bone defect has been formed in cotton-like by electrospinning, the material is divided into a desired size and weight (for embodiment 2 g) by using a pair of tweezers and the like, packed with an aluminum package, and subjected to sterilization treatment. Embodiments of sterilization methods include radiation sterilization (γ rays, electron rays), oxidation ethylene gas sterilization, and high pressure steam sterilization. In the present invention, the radiation sterilization with γ rays is used suitably. In the case where the radiation sterilization with 25 kGy to 35 kGy of γ rays is applied to a sample of PLLA with a molecular weight of 200,000 to 250,000, the molecular weight decreases to 70,000 to 120,000. Fig. 19 shows resultant data of the deceased molecular weight of PLLA in the case where γ rays with a dose of 35 kGy are irradiated to a material for filling a bone defect with a composition of 40TCP (30% by weight of SiV, 40% by weight of TCP, and 30% by weight of PLLA) in an embodiment of the present invention.

### EMBODIMENT

The samples in the embodiment of the present invention were produced by using the materials shown below.
- Silicon-releasing calcium carbonate (SiV): vaterite phase calcium carbonate with a Si content of 2.9% by weight which was prepared by using calcium hydroxide (special grade chemical, a purity of 96% or more, produced by Wako Pure Chemical Industries, Ltd.), methanol (special grade chemical, a purity of 99.8% or more, produced by Wako Pure Chemical Industries, Ltd.), γ-aminopropyltriethoxysilane (SILQUEST A-1100, a purity of 98.5% or more, produced by Momentive Performance Materials Japan Limited Liability Company), and carbon dioxide gas (high purity liquefied carbon dioxide gas, a purity of 99.9%, produced by Taiyo Chemical Industry Co., Ltd.). The details of a method of producing it are disclosed i n Japanese Unexamined Patent Publication No. 2008-100878 (Silicon-eluting calcium carbonate, and its production method).

Fig. 9 shows a structure prognostic chart of SiV, and Fig. 8 shows a SEM photograph of SiV.
- β-tricalcium phosphate (Ca₃(PO₄)₂) : β-TCP-100 produced by Taiyo Chemical Industry Co., Ltd. was used. In the used product (a β-TCP crushed-product), a particle size of 1.7 mm or less in an original product was crushed into about 4 µm.
- PLLA: PURAC produced by Biochem Co., Ltd., PURASORB PL24 Poly (L-lactide), a molecular weight of 200,000 to 300,000 was used.

### 1. Production of a composite

SiV particles and β-TCP particles were added to a polymer melt produced by melting PLLA at 180 °C in a kneader, and then kneaded in the kneader for 12 minutes, and thereafter, cooled and solidified therein to produce a composite of 30SiV, 40β-TCP, and 30PLLA.

### 2. Production of a cotton-like material

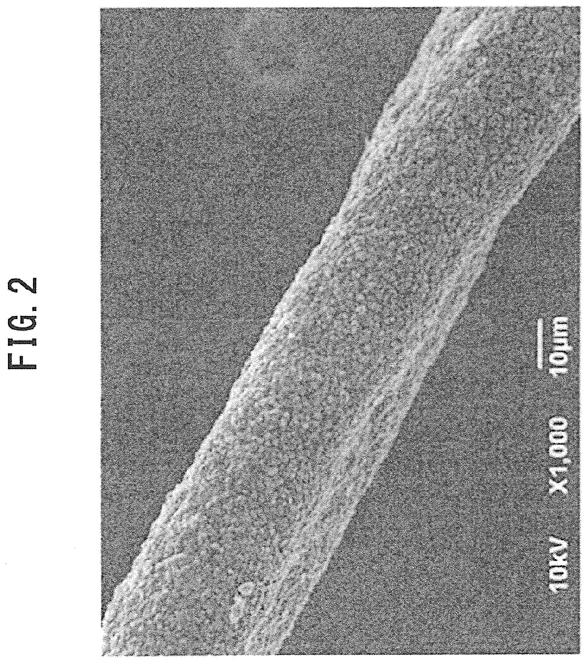

A spinning solution was prepared by dissolving the above composite by chloroform, and then, a cotton-like material formed of biodegradable fibers was produced by spinning the spinning solution by electrospinning.
1) A method of electrospinning
   10% concentration spinning solution for electrospinning was prepared by dissolving the composite with chloroform.
   Thickness of a needle was set to 18G, voltage was set to 25 kV, and a discharging rate of the spinning solution from the nozzle was set to 15 ml/hour. Flying distance from the nozzle to the collector was set to 25 cm. The collector container was filled with ethanol liquid and was configured to receive the electrospun fiber s o that the fiber is deposited therein. As a result of filling the ethanol liquid in the collector, deposited fibers can be prevented from adhering to each other so that it becomes possible to form a cotton-like material with low bulk density.
2) The configuration of a fiber spun by the electrospinning is shown in Fig. 2. The diameter of the spun biodegradable fiber was about 50 µm.

Fig. 3 shows a state where β-TCP particles (average particle diameter is about 3 to 4 µm) and SiV particles (average particle diameter i s about 1 µm) are dispersed almost homogeneously in the PLLA matrix polymer within the fiber having a diameter of 50 µm.

### 3. Characteristics of a cotton-like material

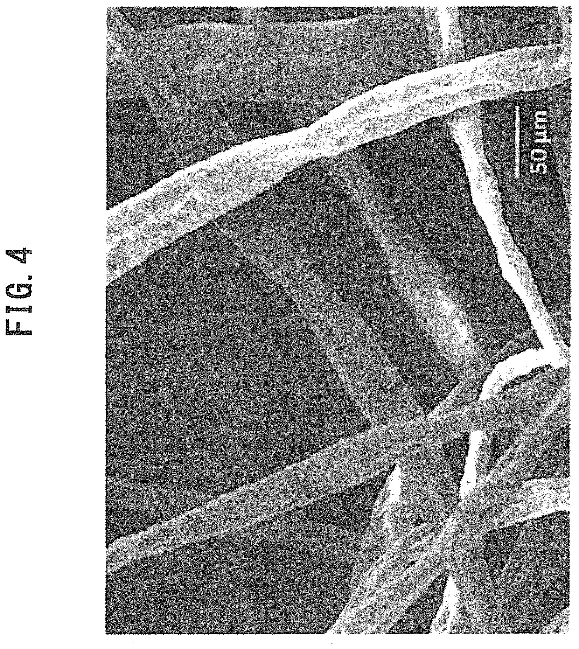

Fig. 4 shows a SEM photograph which shows a cotton-like material in an embodiment of a material for filling a bone defect. Fibers are entangled each other in three dimensional directions to form a cotton-like structure. Those fibers are not adhered each other in a longitudinal direction and are forming a flocculent three dimensional cotton-like structure. The distance between the neighboring fibers which constitute the cotton is about 50 to 200 µm. Average distance is about 50 µm.

Bulk density, compression ratio, and compression recovery ratio of a sample of the cotton-like material of the embodiment were measured in accordance with JIS standard L 1927. Measurement result was that the bulk density was 0.01489 g/cm³, the compression ratio was 52.61%, and the compression recovery ratio was 31.10%.

### 4. Solubility of poly L lactic acid contained in the fibers of the cotton-like material

If the material for filling a bone defect is implanted in a body, the poly L lactic acid polymer constituting the fiber is dissolved and biologically absorbed. The rate differs depending on the difference of the content of the poly L lactic acid contained in the fibers, an amount of an amorphous phase, and the like. Thus, a plurality of samples in the embodiment of the present invention were prepared, and the crystallinity of the plurality of samples was measured by DSC. Further, the multiple samples were immersed in a sodium hydroxide solution. Evaluation and analysis were conducted by observing a change of an appearance and a decrease of molecular weight and a dry weight.

### 1) Method of conducting an experiment

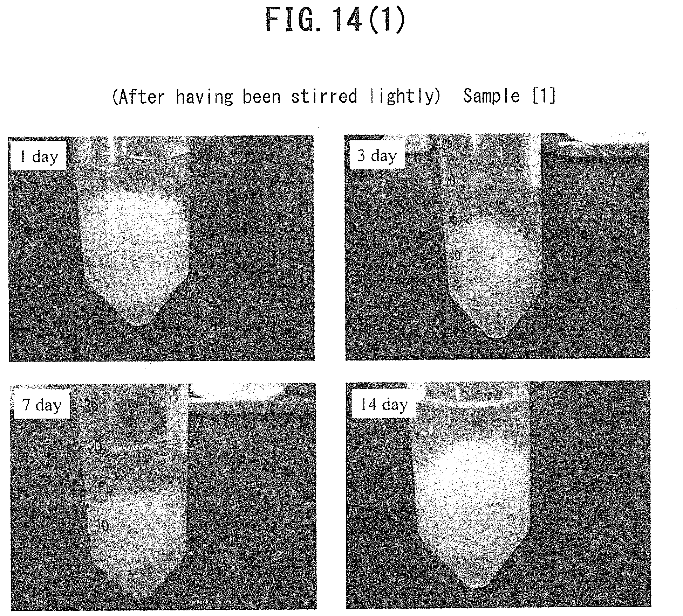

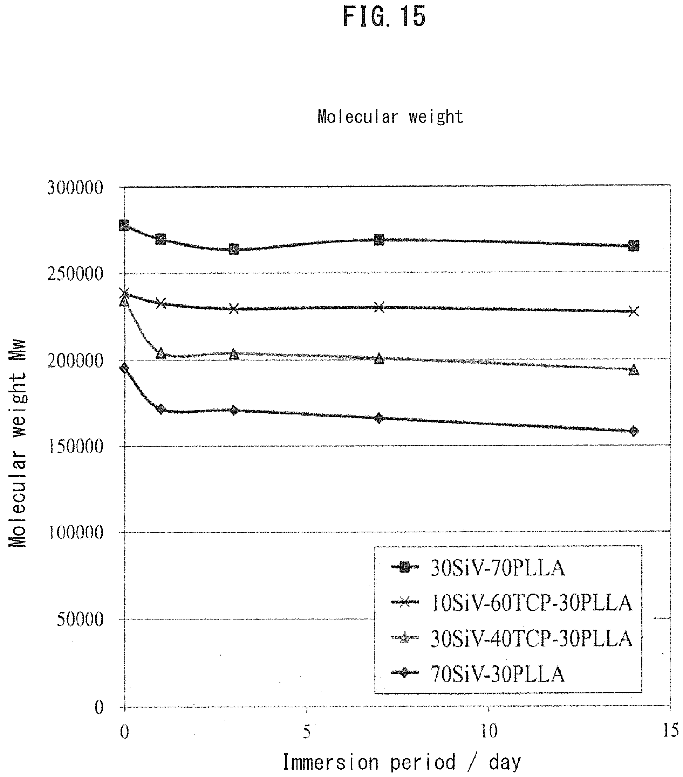

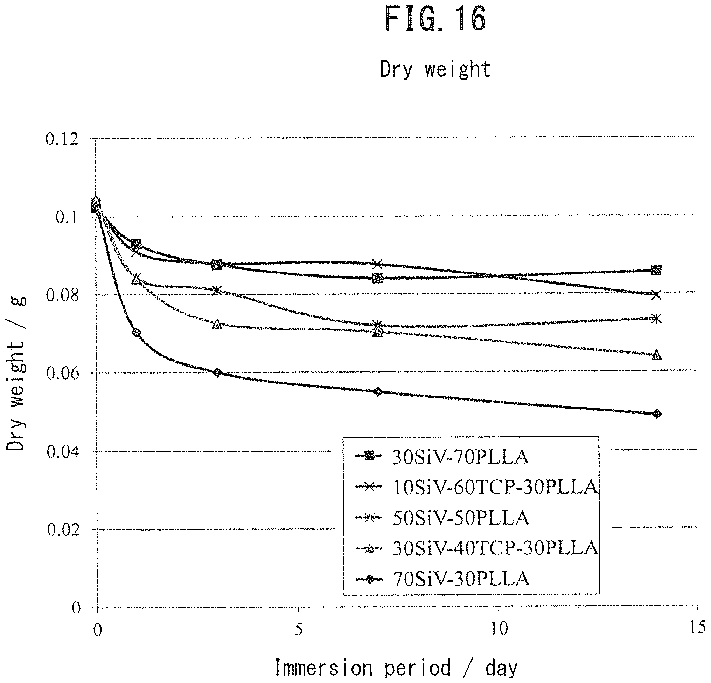

As experiment samples, [1] 30SiV-70PLLA, (not forming part of the invention) [2] 70SiV-30PLLA, (not forming part of the invention) [3] 30SiV-40TCP-30PLLA, [4]10SiV-60TCP-30PLLA, and [5] 50SiV-50PLLA, (not forming part of the invention) each having a different composition weight ratio were produced. The preparation method followed the method described in paragraphs [0038] to [0040]. The crystallinity of the experiment samples [1] to [5] was measured by DSC. The measurement results are shown in Figs. 17(1) and 17(2). The experiment samples [1] to [5] were immersed in a 5 mmol/L sodium hydroxide aqueous solution, and left to stand under a room temperature, and stirred by upturning the container in the morning and at night. Change of appearance and molecular weight (SEM observation) in each of the experiment samples [1] to [5] in the sodium hydroxide aqueous solution were observed at a time after the elapse of one day, three days, seven days, and fourteen days. The results are shown in Figs. 14(1) to 14(5) and Fig 15. Further, the experiment samples [1] to [4] were immersed in a 5 mmol/L sodium hydroxide aqueous solution, and the cotton like material was taken out from the sodium hydroxide aqueous solution at a time of immersing, after one day, three days, seven days, and fourteen days to observe the change of molecular weight and dry weight for each sample. The results are shown in Fig. 16.

### 2) Experiment result

### [Crystallinity]

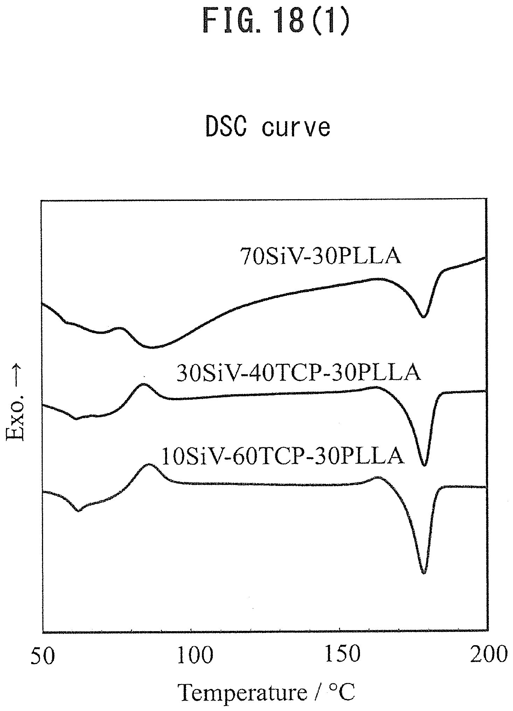

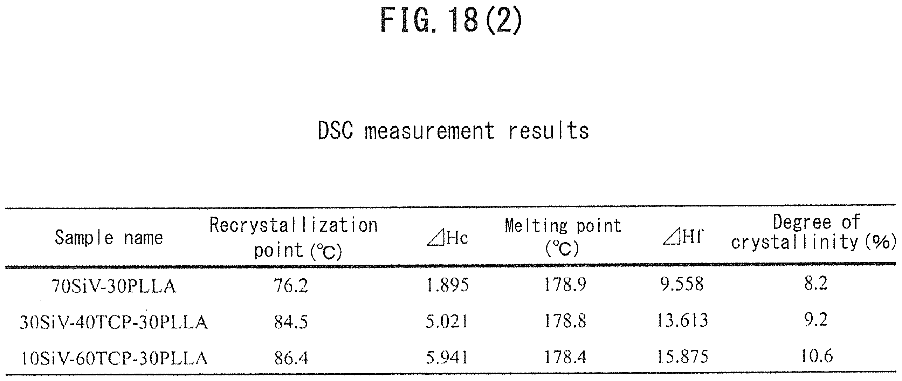

In the DSC measurement result shown in Fig. 17, the crystallinity of raw material PLLA at the beginning was 74.7%. The crystallinity of PLLA in the fibers spun by electrospinning after undergoing the heat kneading greatly decreased to 21.8% or less. It was observed that the crystallinity of PLLA in the spun fibers in the samples ([1] and [5]) with a large PLLA content was higher than that in the samples ([2], [3], and [4]) with a small PLLA content. From the comparison of the three samples ([3], [4], and [5]), each having PLLA content of 30% by weight contained in the spun fibers, it was observed that the crystallinity in the sample which contain TCP and Siv was higher than the crystallinity in the sample which does not contain TCP. Fig. 18 shows the results of the DSC measurement of another samples [2]', [3]', and [4]' which were prepared by the same composition and method as the samples [2], [3], and [4] respectively. Experiment measurement errors are recognized for the data of the crystallinity shown in Fig. 17. In consideration of an expectation that the DSC measurement value of the crystallinity of a sample has an experiment measurement error of ±5% to 10%, it is thought that the crystallinity of each of the samples [2], [3], and [4] is about 75% to 98%, more accurately within a range of about 85% to 95%.

### [Change of a molecular weight]

As shown in the molecular weight measurement in Fig. 15, in the sample [1] which contained 30% by weight of SiV and 70% by weight of PLLA, even though there was a tendency to decrease slightly upon passing fourteen days after the start of the immersion, but a great change was not recognized. In contrast to this, in the sample [2] which contained 70% by weight of SiV and 30% by weight of PLLA and in the sample [3] which contained 30% by weight of SiV, 40% by weight of TCP, and 30% by weight of PLLA, upon passing one day after the start of the immersion, a great decrease of the molecular weight was recognized. Further, in the sample [4] which contained 10% by weight of SiV, 60% by weight of TCP, and 30% by weight of PLLA, upon passing fourteen days after the start of immersion, a moderate decrease tendency of the molecular weight was recognized.

### [Change of a dry weight]

Fig. 16 shows a change (decrease) of the dry weight of the biodegradable fibers due to the elapse of time after the experiment samples [1] to [4] have been immersed in the sodium hydroxide aqueous solution. It was observed that the dry weight of each of the samples [1] to [5] decreased greatly for a short period of time (about one day) after the immersion has been started, and thereafter, the dry weight gradually decreased.

### [Change of appearance]

Fig. 14(1) shows the observation results of a change of appearance of the sample [1] (30SiV-70PLLA) by passing immersion period of 0 day, one day, three days, seven days, and fourteen days after the sample [1] has been immersed in the sodium hydroxide aqueous solution. Even after passing fourteen days since the start of immersion, the three dimensional skeleton of the cotton like structure was still maintained without changing greatly.

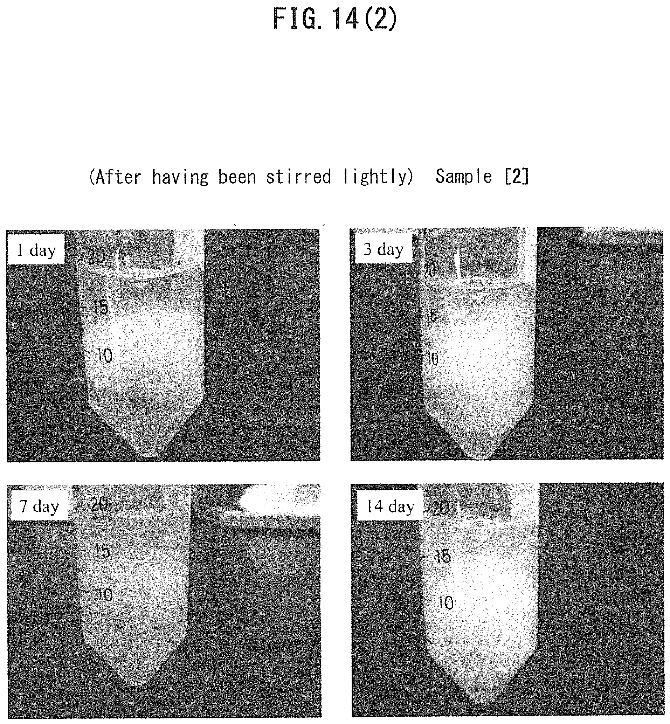

Fig. 14(2) shows the observation results of the sample [2] (70SiV-30PLLA) upon passing immersion period of 0 day, one day, three days, seven days, and fourteen days after the sample [2] has been immersed in the sodium hydroxide aqueous solution. Upon passing three days after the start of immersion, the three dimensional skeleton of the cotton like structure was lost. After fourteen days have elapsed, the cotton like structure did not exist but merely remained as short fibers.

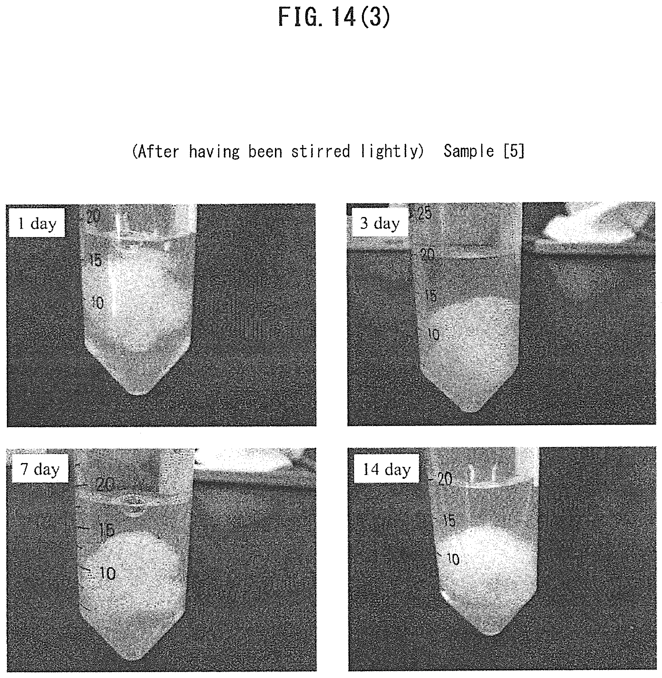

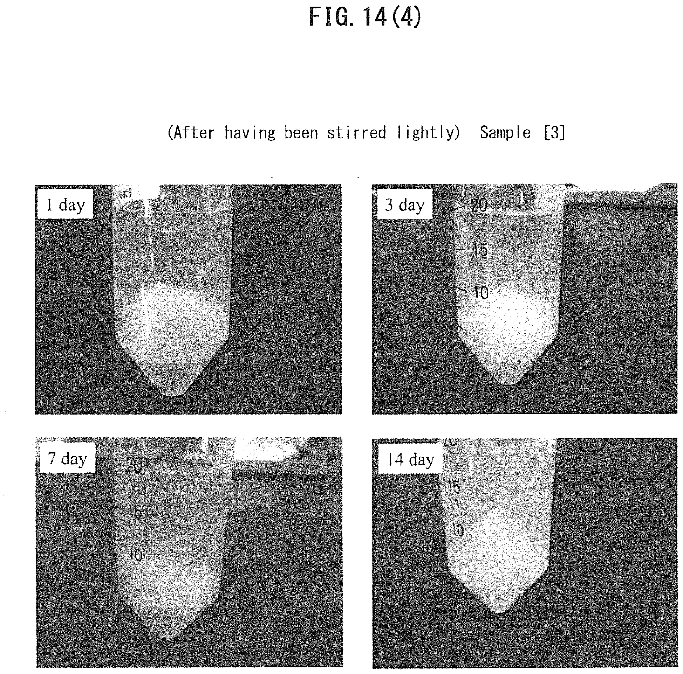

Fig. 14(3) shows the observation results of the sample [5] (50SiV-50PLLA) upon passing immersion period of 0 day, one day, three days, seven days, and fourteen days after the sample [5] has been immersed in the sodium hydroxide aqueous solution. Even after passing fourteen days since start of the immersion, the three dimensional skeleton of the cotton like structure was still maintained without changing greatly. Fig. 14(4) shows the observation results of the sample [3] (30SiV-40TCP-30PLLA) upon passing immersion period of 0 day, one day, three days, seven days, and fourteen days after the sample [3] has been immersed in the sodium hydroxide aqueous solution. Upon passing three days after the start of immersion, the three dimensional skeleton of the cotton has been lost, remaining as short fibers.

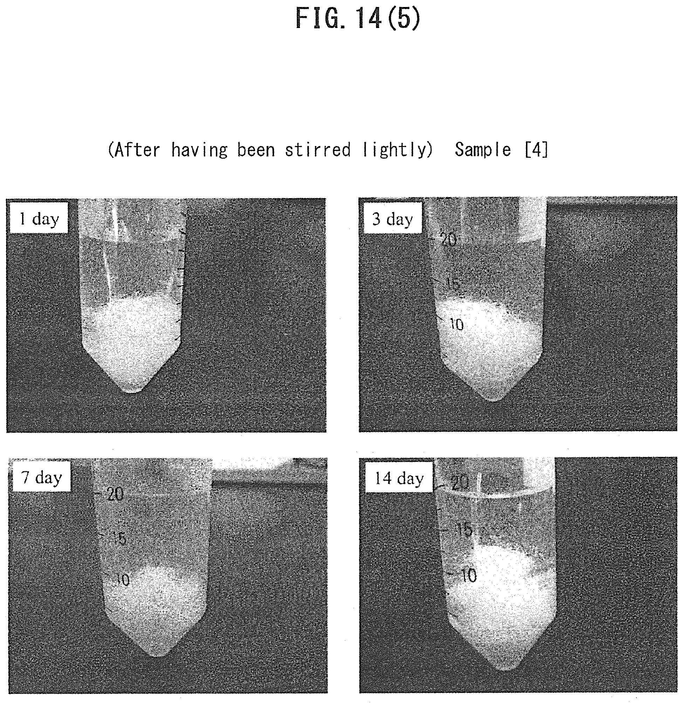

Fig. 14(5) shows the observation results of the sample [4] (10SiV-60TCP-30PLLA) upon passing immersion period of 0 day, one day, three days, seven days, and fourteen days after the sample [4] has been immersed in the sodium hydroxide aqueous solution. After passing fourteen days since the start of the immersion, the three dimensional skeleton of the cotton has be being lost. However, the shape was maintained barely, and the sample [4] remained as short fibers and was floating in the sodium hydroxide aqueous solution.

From the observation of a change of appearance, in the sample with a large PLLA content (the sample [1]), a large change was not found even after passing fourteen days since the sample has been immersed in the sodium hydroxide aqueous solution. Contrary, in the sample with a small PLLA content and a large Siv content (the samples [2] and [3]), a large shape change was observed when fourteen days have passed after the sample has been immersed in the sodium hydroxide aqueous solution. This result almost accords to the change of a molecular weight found at a time when fourteen days have passed after the sample has been immersed in the sodium hydroxide aqueous solution.

### 3. Analysis and evaluation of experimental results

1) As a result of the observation of appearance, it was observed that the sample with a composition of 30% by weight of SiV and 70% by weight of PLLA (the sample [1]) was difficult to decompose in the sodium hydroxide aqueous solution. It is thought that this result comes from the fact that the molecular weight of PLLA of the sample [1] is high (about 270,000) and its crystallinity is high (21.8% according to DSC measurement shown in Fig. 17).
   In 30SiV/40TCP/30PLLA (the sample [3]) which was prepared by mixing 40% by weight of TCP to the composition of sample [1], upon passing one day after the sample has been immersed in the sodium hydroxide aqueous solution, a rapid decrease of molecular weight was observed. In the sample [3], molecular weight is 230,000 and the crystallinity is low (9.1% according to DSC measurement shown in Fig. 17). It is thought that a major reason of this difference comes from the fact that a PLLA content contained in the fibers is 70% by weight in the sample [1], and the PLLA content of the sample [3] is as small as 30% by weight.
2) From the result of the observation of appearance, it was observed that the sample [2] with a composition of 70SiV/30PLLA was decomposed rapidly in the sodium hydroxide aqueous solution. It is thought that this result comes from the fact that in the sample [2], the molecular weight of PLLA is as low as about 200,000 and the crystallinity is low (7.5% according to the DSC measurement shown in Fig. 17).

In 30PLLA/40TCP/30SiV (sample [3]) which was prepared by mixing 40% by weight of TCP to the composition of sample [2], molecular weight of PLLA was about 230,000 and the crystallinity was 9.1% according to DSC measurement shown in Fig. 17. It is thought that this result comes from the fact that occurrence of disturbance in the molecular arrangement order of PLLA due to a reaction between siloxane of SiV and a carboxyl group of PLLA was suppressed. As a result, crystallinity was raised, and decrease of molecular weight and the time of collapse of the three dimensional skeleton of the cotton like material was delayed.

### <Animal experimentation>

Samples of a cotton-like material for filling a bone defect produced in the above embodiment were subjected to sterilization treatment by irradiation of γ rays. Thereafter, the samples were implanted into a femur of a rabbit(sample alone), a spine (bone aspirate is mixed to the sample), and a spine (bone aspirate and an autologous bone are mixed to the sample), and bone formation was evaluated.

Evaluation of X ray visibility immediately after the embedding to the spine was conducted by radiography of a simple X-ray image. Evaluation of bone forming ability was conducted by a CT image and a dye slice. In the preparation method of a dye slice of the femur, a dye slice is prepared in a transverse direction to a bone hole, and a dye slice of a spine was prepared on a sagittal plane. Hematoxylin/eosin was conducted for dyeing.

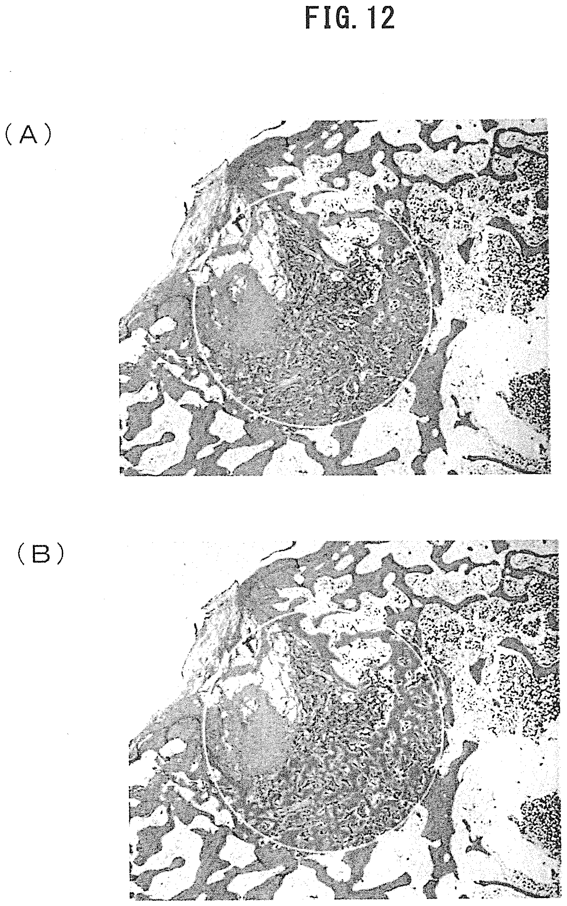

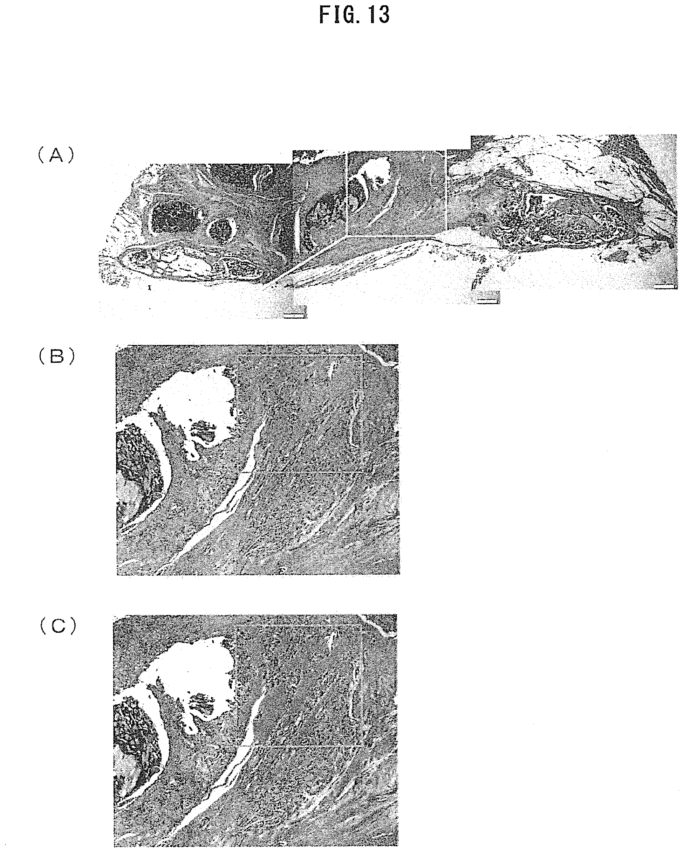

Fig. 11(A) shows radiological data immediately after the implantation to the spine, and Fig. 11(B) shows radiological data after the elapse of twelve weeks after the implantation. Fig. 12 shows histological data and organizational morphometrical data after the elapse of twelve weeks after the implantation to the femur. Fig. 13 shows histological data and organizational morphometrical data after the elapse of twelve weeks after the implantation to the spine.

From the CT image shown in Fig. 11, it is found that upon passing twelve weeks after Sample 1 was implanted in the spine by being mixed with the bone aspirate, a bone was formed at the implanted section.

From the histological data and the organizational morphometrical data shown in Fig. 12, it was confirmed that upon passing twelve weeks after Sample 1 was implanted in the femur, a neonatal bone was formed at a section which occupies 27.1% of a circle-shaped bone hole formed in the femur of the rabbit for the purpose of the experiment.

From the histological data and the organizational morphometrical data shown in Fig. 13, it was confirmed that upon passing twelve weeks after Sample 1 was implanted together with the autologous bone in the spine by being mixed with the bone aspirate, a neonatal bone was formed at an area of 39% of the implanted section.

The material for filling bone defects may be used in a manner that an autologous bone wrapped by the cotton material is filled in the bone defect, other than using the material alone. Because affinity with an autologous bone is high, if autologous bone is filled in a bone defect, bone formation is promoted. Fig. 6 shows a state where an autologous bone is used by being wrapped with the material for filling a bone defect. Silicon released from SiV stimulates osteoblasts of each of an autologous bone and a bone of a defect portion, and promotes bone formation in the portion.

The composite fibers of the material for filling a bone defect are contacted with body fluids in a state where TCP particles and SiV particles are held such that both particles are closely positioned to each other in the matrix polymer. In this state, it is thought that bone formation by the absorption replacement of TCP and bone formation promotion by stimulation of osteoblast by a small amount of silicon are effectively performed in parallel.

A cotton-like material for filling a bone defect formed by biodegradable fibers formed of a composite of poly L lactic acid, calcium phosphate, and silicon-releasing calcium carbonate can be used to fill in the bone defect in a human body such that filling position can be confirmed by X-rays.

In the invention of this application, a bioabsorbable compound such as β-TCP is used as the calcium phosphate. However, calcium phosphate having no bioabsorbability (for example: hydroxyapatite)is the same with β-TCP in a respect that it does not have a silicic acid portion coupled to an amino group. Thus, if a composite of Siv, PLLA, and HAp is prepared by adding a certain amount of hydroxyapatite (HAp) in place of β-TCP, an increase of the amount of amorphous phase caused by the disturbance in molecular order due to occurrence of an amide bond will be suppressed in a similar manner. Therefore, the bio-absorption of the thus-obtained composite can be delayed than a composite of Siv and PLLA. Therefore, it is thought that the invention described in the present application can be basically applied to the composite using HAp to that extent. Specifically, it is possible to prepare a same type of composition as that of the present invention by replacing β-TCP with HAp. For example, it is possible to prepare a composite of SiV of 30% by wight, HAp of 40% by weight, and PLLA of 30 % by weight.

## Claims

1. A method for producing a material for filling a bone defect formed of a mixture of calcium phosphate particles and silicon-releasing calcium carbonate particles added to a biodegradable polymer, comprising the steps of:
- melting PLLA polymer by heating it in a kneader;
- mixing and kneading the calcium phosphate particles and calcium carbonate particles in the PLLA polymer in the kneader such that the PLLA polymer is 30% by weight or more, the calcium phosphate is 40% to 60% by weight and the silicon-releasing calcium carbonate is 10% by weight or more
- cooling the kneaded product under room temperature to be solidified and producing a composite of the silicon-releasing calcium carbonate, the calcium phosphate and the PLLA polymer;
- dissolving the composite by using a solvent to produce a spinning solution
- electrospinning the spinning solution to produce a cotton-like material formed of biodegradable fibers, wherein an amount of amorphous phase of the PLLA polymer in the biodegradable fiber is 75% to 98% by weight
wherein the calcium phosphate particles and the silicon-releasing calcium carbonate particles:
- are both dispersed almost homogeneously in the biodegradable polymer, and
- have both approximately spherical shape, and
- average diameter of the calcium phosphate particles is 3-4 µm, and
- average diameter of the silicon-releasing calcium carbonate particles is about 1 µm.

2. The method of claim 1, wherein the calcium phosphate is β-TCP.

3. The method of any one of the claims 1-2, wherein the calcium carbonate is of vaterite phase.

## Patentansprüche

1. Verfahren zum Herstellen eines Materials zum Füllen eines Knochendefekts gebildet aus einer Mischung aus Calciumphosphatpartikeln und siliziumfreisetzenden Calciumcarbonatpartikeln, die einem biologisch abbaubaren Polymer hinzugefügt werden, umfassend die Schritte:
- Schmelzen von PLLA-Polymer durch Erwärmen in einem Kneter;
- Mischen und Kneten der Calciumphosphatpartikel und Calciumcarbonatpartikel in dem PLLA-Polymer im Kneter, so dass das PLLA-Polymer 30 Gew.-% oder mehr beträgt, das Calciumphosphat 40 bis 60 Gew.-% beträgt und das siliziumfreisetzende Calciumcarbonat 10 Gew.-% oder mehr beträgt.
- Kühlen des gekneteten Produkts bei Raumtemperatur zum Verfestigen und Herstellen eines Verbundes aus dem siliziumfreisetzenden Calciumcarbonat, dem Calciumphosphat und dem PLLA-Polymer;
- Lösen des Verbundes unter Verwendung eines Lösungsmittels zur Herstellung einer Spinnlösung
- Elektrospinnen der Spinnlösung zur Herstellung eines baumwollähnlichen Materials, das aus biologisch abbaubaren Fasern gebildet ist, wobei eine Menge einer amorphen Phase des PLLA-Polymers in der biologisch abbaubaren Faser 75 bis 98 Gew.-% beträgt,
wobei die Calciumphosphatpartikel und die siliziumfreisetzenden Caciumcarbonatpartikel:
- beide nahezu homogen in dem biologisch abbaubaren Polymer dispergiert sind und
- beide eine annähernd sphärische Form aufweisen und
- der durchschnittliche Durchmesser der Calciumphosphatpartikel 3-4 µm beträgt und
- der durchschnittliche Durchmesser der siliziumfreisetzenden Calciumcarbonatpartikel etwa 1 µm beträgt.

2. Verfahren nach Anspruch 1, wobei das Calciumphosphat β-TCP ist.

3. Verfahren nach einem der Ansprüche 1-2, wobei das Calciumcarbonat vom Typ einer Vateritphase ist.

## Revendications

1. Procédé de production d'un matériau pour remplir un défaut osseux formé d'un mélange de particules de phosphate de calcium et de particules de carbonate de calcium libérant du silicium ajoutées à un polymère biodégradable, comprenant les étapes de:
- faire fondre le polymère PLLA en le chauffant dans un pétrin;
- mélanger et pétrir les particules de phosphate de calcium et les particules de carbonate de calcium dans le polymère PLLA dans le pétrin de telle sorte que le polymère PLLA est 30% en poids ou plus, le phosphate de calcium de 40% à 60% en poids et le carbonate de calcium libérant du silicium est de 10% en poids ou plus;
- refroidir le produit pétrissé à température ambiante pour le solidifier et produire un composite du carbonate de calcium libérant du silicium, du phosphate de calcium et du polymère de PLLA;
- dissoudre le composite en utilisant un solvant pour produire une solution de filage;
- électrofiler la solution de filage pour produire un matériau de type coton formé de fibres biodégradables, dans lequel une quantité de phase amorphe du polymère de PLLA dans la fibre biodégradable est comprise entre 75% et 98% en poids;
dans lequel les particules de phosphate de calcium et les particules de carbonate de calcium libérant du silicium:
- sont tous deux dispersés de manière presque homogène dans le polymère biodégradable, et
- ont tous deux une forme approximativement sphérique et
- le diamètre moyen des particules de phosphate de calcium est de 3 à 4 µm, et
- le diamètre moyen des particules de carbonate de calcium libérant du silicium est d'environ 1 µm.

2. Procédé selon la revendication 1, dans lequel le phosphate de calcium est le β-TCP.

3. Procédé selon l'une quelconque des revendications 1 - 2, dans lequel le carbonate de calcium est en phase vatérite.
